# Europäisches Patentamt
# European Patent Office
# Office européen des brevets

(19)

(11) Veröffentlichungsnummer: **0 123 968**
**A1**

(12) # EUROPÄISCHE PATENTANMELDUNG

(21) Anmeldenummer: **84103896.1**

(51) Int. Cl.³: **A 61 M 5/34**

(22) Anmeldetag: **07.04.84**

(30) Priorität: **28.04.83 DE 3315468**

(43) Veröffentlichungstag der Anmeldung: **07.11.84**
Patentblatt 84/45

(84) Benannte Vertragsstaaten: **AT BE CH FR GB IT LI NL SE**

(71) Anmelder: **Almo Erzeugnisse Erwin Busch GmbH,
Grosse Allee 84, D-3548 Arolsen (DE)**

(72) Erfinder: **Hoffmann, Karl, Erpeweg 6, D-3549 Volkmarsen (DE)**

(74) Vertreter: **Walther, Horst, Dipl.-Ing., Wilhelmshöher Allee 275 Postfach 41 01 08, D-3500 Kassel (DE)**

(54) Injektionsspritze, insbesondere Einmalinjektionsspritze.

(57) Injektionsspritze, insbesondere Einmalinjektionsspritze, bestehend aus einem Spritzenzylinder sowie einem an den Zylinderboden sich anschließenden Fortsatz, welcher die Kanüle trägt, wobei das eine Ende (5a) der Kanüle (5) bis zum Zylinderboden (2) reicht.

ACTORUM AG

## Dipl.-Ing. HORST WALTHER

Zugelassener Vertreter beim Europäischen Patentamt

PATENTANWALT

0123968

Postscheck-Kto. 149359-602 Ffm.

Bankkonten in Kassel:
Raiffeisenbank 6573355 (BLZ 52060515)
Dresdner Bank 425498300 (BLZ 52080080)

W.-Germany
3500 Kassel-Wilh.
Wilhelmshöher Allee 275
Postfach 410108
Telefon 0561 / 38714

Dipl.-Ing. H. Walther · 35 Kassel · Wilhelmshöher Allee 275

Tag:

22.03.1984

843/10562

Firma
Almo Erzeugnisse
Erwin Busch GmbH

Große Allee 84

3548 Arolsen

Injektionsspritze, insbesondere Einmalinjektionsspritze

Die Erfindung betrifft eine Injektionsspritze,
insbesondere eine Einmalinjektionsspritze, bestehend aus einem Spritzenzylinder sowie einem an
den Zylinderboden angesetzten Fortsatz, welcher die
Kanüle als abnehmbaren Teil trägt.

Es sind Injektionsspritzen, insbesondere sogenannte
Einmalinjektionsspritzen aus Kunststoff bekannt,
bei denen am Zylinderboden ein männlicher Kegel angebracht ist, der eine größere Bohrung aufweist.
Auf diesen Kegel wird die mit einem weiblichen
Kegelansatz versehene Kanüle geschoben.

- 2 -

Diese Ausbildung hat aber den Nachteil, daß nach dem Spritzen einer medikamentösen Flüssigkeit, immer noch ein gewisses Restvolumen verbleibt, das sich in der relativ großen Durchbohrung des Fortsatzes ansammelt. Dieses Restvolumen ist deshalb nicht verwertbar, weil der im Spritzenzylinder verschiebbare Kolben nur bis zu dem Zylinderboden reicht und mithin alle Flüssigkeit die sich in Hohlräumen ansammelt, die sich an den Zylinderboden anschließen, für die Verwertung verloren ist.

Der Erfindung liegt daher die Aufgabe zugrunde eine Injektionsspritze, insbesondere eine Einmalinjektionsspritze zu schaffen, bei der ein Restvolumen nicht übrig bleibt, wenn man einmal von dem geringen Kanülenvolumen absieht.

Nach der Erfindung ist die Injektionsspritze dadurch gekennzeichnet, daß das eine Ende der Kanüle bis zum Zylinderboden reicht. Eine besonders vorteilhafte Ausführungsform ist dabei dadurch gekennzeichnet, daß der Fortsatz eine Einsatzbohrung aufweist, in die die Kanüle mit ihrem endseitig angeordneten Einsatzstück reicht.

Die Einsatzbohrung und das Einsatzstück sind dabei konisch ausgebildet.

Dadurch ist erreicht, daß sich ein Restvolumen im Fortsatz nicht ansammeln kann, da der Kolben praktisch bis zur Kanüle selbst verschiebbar ist, wenn er seine Endstellung erreicht hat.

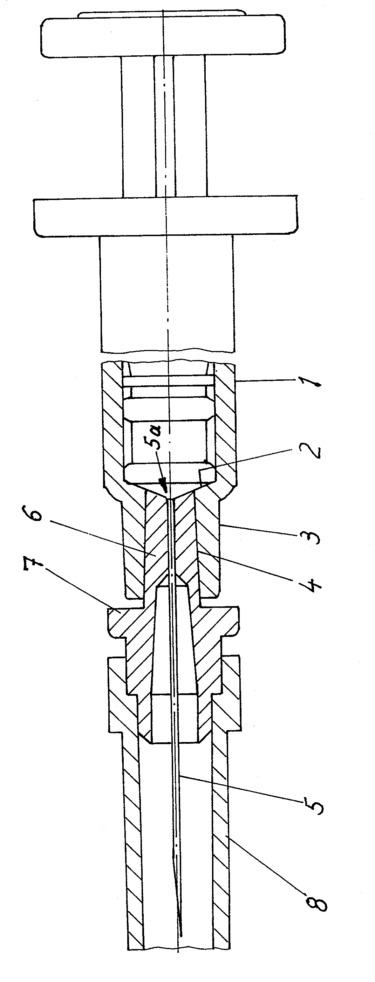

In der Zeichnung ist eine beispielsweise Ausführungsform dargestellt.

Mit 1 ist der Spritzenzylinder bezeichnet, der den Zylinderboden 2 aufweist. Am Zylinderboden ist ein Fortsatz 3 angesetzt. Dieser Fortsatz 3 besitzt eine Einsatzbohrung 4, in die die Kanüle 5 mit ihrem endseitig angebrachten Einsatzstück 6 eingesetzt ist. Das Einsatzstück weist dabei zugleich auch einen Träger 7 auf, auf den die Schutzhülse 8 aufgeschoben wird. Die Einsatzbohrung 4 und das Einsatzstück 6 sind konisch ausgebildet.

Bei dieser Ausbildung einer Injektionsspritze reicht die Kanüle 5, mit ihrem einen Ende 5a, bis zum Zylinderboden 2. Dadurch verbleibt beim Spritzen lediglich das geringe Volumen der Kanüle.

- 4 -

Das sich sonst bei den bekannten Spritzen in der
größeren Durchbohrung des Fortsatzes ansammelnde
Restvolumen ist damit vermieden.

- Ansprüche -

Firma Almo Erzeugnisse Erwin Busch GmbH
Große Allee 84, 3548 Arolsen

A n s p r ü c h e

1. Injektionsspritze, insbesondere Einmalinjektionsspritze, bestehend aus einem Spritzenzylinder
sowie einem an den Zylinderboden sich anschliessenden Fortsatz, welcher die Kanüle als abnehmbaren Teil trägt

d a d u r c h  g e k e n n z e i c h n e t, daß

das eine Ende (5a) der Kanüle (5) bis zum Zylinderboden (2) reicht.

2. Injektionsspritze nach Anspruch 1

d a d u r c h  g e k e n n z e i c h n e t, daß

der Fortsatz (3) eine Einsatzbohrung (4) aufweist, in die die Kanüle mit ihrem endseitig
angeordneten Einsatzstück (6) eingesetzt ist.

3. Injektionsspritze nach Anspruch 1

d a d u r c h  g e k e n n z e i c h n e t, daß

die Einsatzbohrung (4) und das Einsatzstück (6)
konisch ausgebildet sind.

4. Injektionsspritze nach Anspruch 1

d a d u r c h  g e k e n n z e i c h n e t , daß

das Einsatzstück (6) einen Träger (7) für die
Schutzhülse (8) aufweist.

0123968

Europäisches
Patentamt

**EUROPÄISCHER RECHERCHENBERICHT**

Nummer der Anmeldung

EP 84 10 3896

| | **EINSCHLÄGIGE DOKUMENTE** | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
| X | FR-A-2 357 261 (BECTON, DICKINSON AND CO.) <br> * Seite 1, Zeile 33 - Seite 2, Zeile 2; Seite 2, Zeile 33 - Seite 5, Zeile 6; Zeichnungen 1-3,6 * | 1-4 | A 61 M 5/34 |
| | --- | | |
| X | US-A-4 249 530 (MILLET) <br> * Spalte 1, Zeile 59 - Spalte 2, Zeile 5; Spalte 3, Zeilen 19-53; Zeichnungen 3,4 * | 1-4 | |
| | --- | | |
| X | FR-A- 574 278 (GENTILE) <br> * Seite 1, Zeilen 35-47; Zeichnung * | 1-3 | |
| | ----- | | RECHERCHIERTE SACHGEBIETE (Int. Cl. ³) |
| | | | A 61 M |

Der vorliegende Recherchenbericht wurde für alle Patentansprüche erstellt.

| Recherchenort <br> DEN HAAG | Abschlußdatum der Recherche <br> 02-08-1984 | Prüfer <br> KNAUER F.E. |
|---|---|---|